# Europäisches Patentamt

# European Patent Office

# Office européen des brevets

(11) Publication number : **0 541 333 A1**

# EUROPEAN PATENT APPLICATION

(21) Application number : **92310064.8**

(22) Date of filing : **03.11.92**

(51) Int. Cl.⁵ : **C12P 7/40,** C12P 13/04, C07F 9/38

(30) Priority : **06.11.91 US 788648**
**06.11.91 US 788640**
**06.11.91 US 788683**

(43) Date of publication of application :
**12.05.93 Bulletin 93/19**

(84) Designated Contracting States :
**PT**

(71) Applicant : **E.I. DU PONT DE NEMOURS AND COMPANY**
**1007 Market Street**
**Wilmington Delaware 19898 (US)**

(72) Inventor : **Anton, David Leroy**
**201 West Pembrey Drive**
**Wilmington, Delaware 19803 (US)**
Inventor : **Di Cosimo, Robert**
**2817 Fawkes Drive**
**Wilmington, Delaware 19808 (US)**
Inventor : **Porta, Earnest William**
**104 Fox Brook Drive**
**Landenberg, Pennsylvania 19350 (US)**

(74) Representative : **Fisher, Adrian John et al**
**CARPMAELS & RANSFORD 43 Bloomsbury Square**
**London WC1A 2RA (GB)**

(54) Hydrogenation of enzymatically-produced glyoxylic acid/aminomethylphosphonic acid mixtures.

(57)    The invention provides a process for the production of N-(phosphonomethyl)glycine, also known as glyphosate. The process comprises hydrogenating a mixture of glyoxylic acid and aminomethylphosphonic acid, the mixture having been enzymatically prepared *in situ* by the reaction of glycolic acid and oxygen in an aqueous solution containing aminomethylphosphonic acid and the enzymes glycolate oxidase and catalase.

EP 0 541 333 A1

## BACKGROUND OF THE INVENTION

### 1. Field of the Invention:

This invention relates to the preparation of N-(phosphonomethyl)glycine by the hydrogenation of mixtures produced by the reaction of glycolic acid and oxygen in an aqueous solution containing aminomethylpbosphonic acid (AMPA) and the enzymes glycolate oxidase ((S)-2-hydroxy-acid oxidase, EC 1.1.3.15) and catalase (EC 1.11.1.6). N-(phosphonomethyl)glycine is a broad-spectrum, postemergent herbicide useful in controlling the growth of a wide variety of plants.

### 2. Description of the Related Art:

Glycolate oxidase, an enzyme commonly found in leafy green plants and mammalian cells, catalyzes the oxidation of glycolic acid to glyoxylic acid, with the concomitant production of hydrogen peroxide:

$$HOCH_2CO_2H + O_2 \rightarrow OCHCO_2H + H_2O_2$$

N. E. Tolbert et al., J. Biol. Chem., Vol. 181, 905-914 (1949) first reported an enzyme, extracted from tobacco leaves, which catalyzed the oxidation of glycolic acid to formic acid and $CO_2$ via the intermediate formation of glyoxylic acid. The addition of certain compounds, such as ethylenediamine, limited the further oxidation of the intermediate glyoxylic acid. The oxidations were carried out at a pH of about 8, typically using glycolic acid concentrations of about 3-40 mM (millimolar). The optimum pH for the glycolate oxidation was reported to be 8.9. Oxalic acid (100 mM) was reported to inhibit the catalytic action of the glycolate oxidase. Similarly, K. E. Richardson and N. E. Tolbert, J. Biol. Chem., Vol. 236, 1280-1284 (1961) showed that buffers containing tris(hydroxymethyl)aminomethane (TRIS) inhibited the formation of oxalic acid in the glycolate oxidase catalyzed oxidation of glycolic acid. C. O. Clagett, N. E. Tolbert and R. H. Burris, J. Biol. Chem., Vol. 178, 977-987 (1949) reported that the optimum pH for the glycolate oxidase catalyzed oxidation of glycolic acid with oxygen was about 7.8-8.6, and the optimum temperature was 35-40°C.

I- Zelitch and S. Ochoa, J. Biol. Chem., Vol. 201, 707-718 (1953), and J. C. Robinson et al., J. Biol. Chem., Vol. 237, 2001-2009 (1962), reported that the formation of formic acid and $CO_2$ in the spinach glycolate oxidase-catalyzed oxidation of glycolic acid resulted from the nonenzymatic reaction of $H_2O_2$ with glyoxylic acid. They observed that addition of catalase, an enzyme that catalyzes the decomposition of $H_2O_2$, greatly improved the yields of glyoxylic acid by suppressing the formation of formic acid and $CO_2$. The addition of FMN (flavin mononucleotide) was also found to greatly increase the stability of the glycolate oxidase.

N. A. Frigerio and H. A. Harbury, J. Biol. Chem., Vol. 231, 135-157 (1958) have reported on the preparation and properties of glycolic acid oxidase isolated from spinach. The purified enzyme was found to be very unstable in solution; this instability was ascribed to the relatively weak binding of flavin mononucleotide (FMN) to the enzyme active site, and to the dissociation of enzymatically active tetramers and/or octamers of the enzyme to enzymatically-inactive monomers and dimers, which irreversibly aggregate and precipitate. The addition of FMN (flavin mononucleotide) to solutions of the enzyme greatly increased its stability, and high protein concentrations or high ionic strength maintained the enzyme as octamers or tetramers.

There are numerous other references to the oxidation of glycolic acid catalyzed by glycolic acid oxidase. The isolation of the enzyme (and an assay method) are described in the following references: I. Zelitch, Methods in Enzymology, Vol. 1, Academic Press, New York, 1955, p. 528-532 (from spinach and tobacco leaves), M. Nishimura et al., Arch, Biochem, Biophys., Vol. 222, 397-402 (1983) (from pumpkin cotyledons), H. Asker and D. Davies, Biochim. Biophys. Acta, Vol. 761, 103-108 (1983) (from rat liver), and M. J. Emes and K. H. Erismann, Int. J. Biochem., Vol. 16, 1373-1378 (1984) (from Lemna Minor L). The structure of the enzyme has also been reported: E. Cederlund et al., Eur.J. Biochem., Vol. 173, 523-530 (1988), and Y. Lindquist and C. Branden, J. Biol. Chem.,Vol. 264, 3624-3628, (1989).

Numerous methods are known for preparing N-(phosphonomethyl)glycine from aminomethylphosphonic acid and glyoxylic acid. One such method, described in Rogers et al., European Patent Application 186,648, involves condensation of glyoxylic acid or a salt thereof with aminomethylphosphonic acid or a salt thereof to form an intermediate product, generally regarded as an aldimine (Schiff base), which without isolation is reduced, as by catalytic hydrogenation, to N-(phosphonomethyl)glycine. A second method, described in Gaertner, U.S. Patent 4,094,928, isolates these same intermediate carbonylaldiminomethanephosphonates by the reaction of glyoxylic acid esters with aminomethylphosphonate esters in a non-aqueous solvent; after azeotropic distillation of water and removal of the solvent, the carbonylaldiminomethanephosphonate ester is reduced and the ester groups hydrolyzed to produce N-(phosphonomethyl)glycine.

The above routes to N-(phosphonomethyl)glycine suffer in that glyoxylic acid is a rather costly starting material, and other less expensive routes to the desired material are practiced. Existing methods for the preparation of glyoxylic acid, such as hydrolysis of a dihaloacetic acid, electrolytic reduction of oxalic acid, oxidation of glyoxal, catalytic oxidation of ethylene or acetaldehyde, and ozonolysis of maleic acid, its esters or anhydride, present one or more difficulties in practice, e.g. costly separation/purification steps, low yields, or large waste streams. The method described in Gaertner is also disadvantageous in that it requires several additional steps (with corresponding losses in yield), and the unnecessary isolation of an intermediate.

Another method for the synthesis of N-(phosphonomethyl)glycine, disclosed in Kleiner, U.S. Pat. 4,670,191, comprises the reaction of aminomethylphosphonic acid or a salt thereof with about two molar equivalents of glyoxylic acid in aqueous medium. The excess glyoxylic acid evidently functions as a reducing agent, converting an intermediate glyoxylic acid-aminomethylphosphonic acid reaction product to the desired N-(phosphonomethyl)glycine, and is itself oxidized to one or more by-products, including $CO_2$. Similarly, Fields et al., in U.S. Pat. 4,851,159 prepare N-(phosphonomethyl)glycine by heating an N-acylaminomethylphosphonic acid with glyoxylic acid or a derivative thereof. The mole ratio of the glyoxylic to the N-acylamino component is preferably 2 to 1; otherwise at smaller ratios the yield suffers.

The Kleiner and Fields et al. processes entail the disadvantages of not only employing relatively expensive glyoxylic acid but of employing it as a sacrificial reductant (ca. one mole of glyoxylate employed as reductant for every mole of N-(phosphonomethyl)glycine produced) as well as the condensing agent for the amino-(or N-acylamino) methylphosphonic acid.

## SUMMARY OF THE INVENTION

The process for preparing N-(phosphonomethyl)glycine according to the present invention involves hydrogenating a mixture, wherein the mixture is enzymatically produced by reacting glycolic acid and oxygen in an aqueous solution containing aminomethylphosphonic acid (AMPA) and the enzymes glycolate oxidase and catalase. It should be appreciated for purposes of this invention that the mixtures produced by enzymatic oxidation of glycolic acid in the presence aminomethylphosphonic acid inherently result in a distribution of oxidation by-products in addition to the desired glyoxylic acid component (including by way of example but not limited thereto, oxalate, formate, and carbon dioxide). Also present in such mixtures will be unreacted glycolate as well as various additives such as flavin mononucleotide (hereinafter referred to as FMN) or the like, all of which may or may not influence the desired subsequent hydrogenation reaction (again by way of example, but not limited thereto, it has been found that both formate and FMN lower the recovered carbon balance when present during the hydrogenation of glyoxylic acid in the presence AMPA). Thus the present invention further provides for the removal and recovery of the enzymes from the solution produced as a result of enzymatic oxidation as well as the optional removal of FMN prior to the hydrogenation step.

Thus the present invention provides an improved process for preparing N-(phosphonomethyl)glycine comprising the step of reducing a mixture of glyoxylic acid and aminomethylphosphonic acid by hydrogenation; said mixture being enzymatically generated *in situ* in an aqueous solution by incorporating into the aqueous solution glycolic acid, a first catalyst adapted to catalyze the oxidation of glycolic acid with oxygen to glyoxylic acid and hydrogen peroxide, and a second catalyst adapted to catalyze the decomposition of hydrogen peroxide, adjusting the pH of the solution to between 6 and about 10, contacting the solution with a source of oxygen at an effective temperature and sufficient time to convert at least a portion of the glycolic component to the glyoxylic component in the presence of aminomethylphosphonic acid, and ceasing contacting the solution with oxygen prior to the reducing step.

Preferably, the catalysts are enzymatic; more preferably the first enzyme is glycolate oxidase ((S)-2-hydroxy-acid oxidase, EC 1.1.3.15) and the second enzyme is catalase (EC 1.11.1.6). After the contacting of the solution with $O_2$ in the presence of the catalysts/enzymes is ceased, the catalysts/enzymes are removed, as by filtration or centrifugation, before the solution is subjected to reducing conditions for the production of N-(phosphonomethyl)glycine.

Thus, by obviating the need to prepare glyoxylic acid in a separate step, the present invention provides for a more efficient and economic process for the production of N-(phosphonomethyl)glycine.

It is an object of this invention to provide an improved process for the production of N-(phosphonomethyl)glycine by reduction of mixtures of glyoxylic acid and aminomethylphosphonic acid which avoids the need to separately prepare glyoxylic acid.

Another object is to provide such a process wherein glyoxylic acid is enzymatically generated *in situ* in the presence of aminomethylphosphonic acid from a readily available precursor thereof, namely glycolic acid, thereby affording a more efficient and economic process for the production of N-(phosphonomethyl)glycine.

## DETAILED DESCRIPTION OF THE INVENTION

The improved process for the production of N-(phosphonomethyl)glycine according to the present invention involves the reduction of a mixture containing glyoxylic acid (or a suitable derivative thereof) with aminomethylphosphonic acid (AMPA) (or a suitable derivative thereof). Preferably, the mixture is prepared by catalytically oxidizing a glycolic acid component or a suitable salt thereof by contacting the glycolic by contacting the glycolic acid component with a source of molecular oxygen in the presence of AMPA and a catalyst effective to catalyze the reaction of glycolic acid with $O_2$ to form glyoxylic acid. One such catalyst is a naturally-occurring enzyme glycolate oxidase (EC 1.1.3.15), also known as glycolic acid oxidase, which is capable of catalyzing the reaction to produce glyoxylic acid in high yields at high glycolic acid conversions in aqueous media under mild conditions of pH and temperature, i.e.,

$$HOCH_2CO_2H + O_2 \rightarrow OCHCO_2H + H_2O_2$$

The catalytic oxidation of glycolic acid or a suitable salt thereof is conveniently carried out by contacting the glycolic acid with a source of molecular oxygen in the presence of an enzyme catalyst which catalyzes the reaction of glycolic acid with $O_2$ to form glyoxylic acid. One such catalyst is the enzyme glycolate oxidase (EC 1.1.3.15), also known as glycolic acid oxidase. Glycolate oxidase may be isolated from numerous sources well-known to the art. The glycolate oxidase used in the reaction should be present in an effective concentration, usually a concentration of about 0.01 to about 1000 IU/mL, preferably about 0.1 to about 4 IU/mL. An IU (International Unit) is defined as the amount of enzyme that will catalyze the transformation of one micromole of substrate per minute. A procedure for the assay of this enzyme is found in I. Zelitch and S. Ochoa, J. Biol. Chem., Vol. 201, 707-718 (1953). This method is also used to assay the activity of recovered or recycled glycolate oxidase.

Although the enzyme-catalyzed reaction of glycolic acid with oxygen is well known, high selectivities to glyoxylic acid have not been previously obtained, and there are no previous reports of performing the enzymatic oxidation of glycolic acid in the presence of aminomethylphosphonic acid (AMPA). A previous application, International Publication Number WO 91/05868, May 2, 1991, "Production of Glyoxylic Acid by Enzymatic Oxidation of Glycolic Acid", described a process for the enzymatic conversion of glycolic acid to glyoxylic acid in the presence of oxygen, an amine buffer, and the soluble enzymes glycolate oxidase and catalase. This process demonstrated the unexpected synergistic effect of using both catalase (to destroy byproduct hydrogen peroxide) and an amine buffer capable of forming a chemical adduct with the glyoxylic acid produced (limiting its further oxidation). Neither the separate addition of catalase nor an amine buffer was found to produce the high selectivity observed when both were present, and the almost quantitative yields of glyoxylic acid obtained were more than expected from a simple additive effect of using catalase or amine buffer alone.

Improvements in the yields of glyoxylate produced by the formation of an oxidation-resistant complex of glyoxylate and an amine buffer (via the formation of an N-substituted hemiaminal and/or imine) were found to be dependent on the pKa of the protonated amine buffer. The result of oxidizing aqueous solutions of glycolic acid (0.25 M) in the presence of an amine buffer (0.33 M, pH 8.3), glycolate oxidase (0.5 IU/mL), catalase (1,400 IU/mL), and FMN (0.01 mM) at 30°C, and under 1 atm of oxygen for 24 h, are listed in the table below, along with reactions performed using two buffers not expected to complex with glyoxylate (phosphate and bicine):

| Buffer (pKa) | % oxalate | %glyoxylate | %glycolate | %formate |
|---|---|---|---|---|
| ethylenediamine (6.85, 9.93) | 6.8 | 85.5 | 0.8 | 2.4 |
| TRIS (8.08) | 1.1 | 81.0 | 2.8 | 12.0 |
| methylamine (10.62) | 1.0 | 53.9 | 39.8 | 5.1 |
| ethanolamine (9.50) | 1.8 | 69.6 | 4.81 | 24.5 |
| ammonium chloride (9.24) | 1.1 | 39.9 | 37.7 | 18.9 |
| isopropanolamine (9.43) | 2.0 | 60.0 | 4.8 | 37.4 |
| bicine (8.30) | 1.0 | 24.9 | 25.6 | 43.8 |
| phosphate (2.15, 7.10, 12.3) | 0.7 | 24.5 | 52.4 | 21.2 |

Of the amine buffers examined, amines with a pKa approximately equal to or lower than the pH of the reaction mixture (i.e., ethylenediamine and TRIS) produced much higher yields of glyoxylate (and low formate and oxalate production) than amine buffers whose pKas were higher than the pH at which the reaction was performed. These results are consistent with the expectation that an unprotonated amine may be necessary to form an oxidation-resistant N-substituted hemiaminal and/or imine complex with glyoxylate; an amine buffer whose pKa is much higher than the pH of the reaction mixture would be present predominantly as the protonated ammonium ion in the reaction mixture, and therefore be less likely to form such complexes with glyoxylate.

The pKa of the protonated amine of aminomethylphosphonic acid (AMPA) is reported to be 10.8 (Lange's Handbook of Chemistry, J. A. Dean, Ed., McGraw-Hill, New York, 1979, 12th Edition), therefore it was unexpected that the addition of AMPA to enzymatic oxidations of glycolic acid within the pH range of 7 to 9 would result in high yields of glyoxylic acid. The accompanying Examples illustrate that yields of glyoxylic acid as high as 92% have been attained using this amine. In addition to the unexpected high yields of glyoxylic acid obtained, the use of AMPA also results in an improvement in recovery of glycolate oxidase and catalase activity when compared to reactions run in the absence of added AMPA (Example 13). Recovery of catalyst for recycle is usually required in processes utilizing enzyme catalysts, where catalyst cost makes a significant contribution to the total cost of manufacture.

Optimal results in the use of glycolate oxidase as a catalyst for the oxidative conversion of glycolic acid to glyoxylic acid are obtained by incorporating into the reaction solution a catalyst for the decomposition of hydrogen peroxide. One such peroxide-destroying catalyst which is effective in combination with glycolate oxidase is the enzyme catalase (E.C. 1.11.1.6).

Catalase catalyzes the decomposition of hydrogen peroxide to water and oxygen, and it is believed to improve yields of glyoxylic acid in the present process by accelerating the decomposition of the hydrogen peroxide produced along with glyoxylic acid in the glycolate oxidase-catalyzed reaction of glycolic acid with $O_2$. The concentration of catalase should be 50 to 50,000 IU/mL, preferably 500 to 15,000 IU/mL. It is preferred that the catalase and glycolate oxidase concentrations be adjusted within the above ranges so that the ratio (measured in IU for each enzyme) of catalase to glycolate oxidase is at least about 250:1.

Another optional but often beneficial ingredient in the reaction solution is flavin mononucleotide (FMN), which is generally used at a concentration of 0.0 to about 2.0 mM, preferably about 0.01 to about 0.2 mM. It is believed the FMN increases the productivity of the glycolate oxidase, by which is meant the amount of glycolic acid converted to glyoxylic acid per unit of enzyme increases. It is to be understood that the concentration of added FMN is in addition to any FMN present with the enzyme, because FMN is often also added to the enzyme during the preparation of the enzyme. The structure of FMN and a method for its analysis is found in K. Yagai, Methods of Biochemical Analysis, Vol. X Interscience Publishers, New York, 1962, p. 319-355, which is hereby included by reference.

Glycolic acid (2-hydroxyacetic acid) is available commercially. In the present reaction its initial concentration is in the range of 0.10 M to 2.0 M, preferably between 0.25 M and 1.0 M. It can be used as such or as a compatible salt thereof, that is, a salt that is water-soluble and whose cation does not interfere with the desired conversion of glycolic acid to glyoxylic acid, or the subsequent reaction of the glyoxylic acid product with the amino-methylphosphonic acid to form N-(phosphonomethyl)glycine. Suitable and compatible salt-forming cationic groups are readily determined by trial. Representative of such salts are the alkali metal, alkaline earth metal, ammonium, substituted ammonium, phosphonium, and substituted phosphonium salts.

The conversion of glycolic acid to glyoxylic acid is conveniently and preferably conducted in aqueous media. Aminomethylphosphonic acid (AMPA), or a suitable salt thereof, is added to produce a molar ratio of AMPA/glycolic acid (starting amount) in the range of from 0.01/1.0 to 3.0/1.0, preferably from 0.25/1.0 to 1.05/1.0. After combining AMPA and glycolic acid in an aqueous solution, the pH of the resulting mixture is adjusted to a value between 6 and 10, preferably between 7.0 and 9.0. Within this pH range, the exact value may be adjusted to obtain the desired pH by adding any compatible, non-interfering base, including alkali metal hydroxides, carbonates, bicarbonates and phosphates. The pH of the reaction mixture decreases slightly as the reaction proceeds, so it is often useful to start the reaction near the high end of the maximum enzyme activity pH range, about 9.0 - 8.5, and allow it to drop during the reaction. The pH can optionally be maintained by the separate addition of a non-interfering inorganic or organic buffer, since enzyme activity varies with pH.

It is understood that glycolic and glyoxylic acids are highly dissociated in water, and at pH of between 6 and 10 are largely if not substantially entirely present as glycolate and glyoxylate ions. It will also be appreciated by those skilled in the art that glyoxylic acid (and its conjugate base, the glyoxylate anion) may also be present as the hydrate, e.g. $(HO)_2CHCOOH$ and/or as the hemiacetal, $HOOCCH(OH)OCH(OH)COOH$, which compositions and their anionic counterparts are equivalent to glyoxylic acid and its anion for the present purpose of being suitable reactants for N-(phosphonomethyl)glycine formation.

Oxygen ($O_2$), the oxidant for the conversion of the glycolic acid to glyoxylic acid, may be added as a gas to the reaction by agitation of the liquid at the gas-liquid interface or through a membrane permeable to oxygen. It is believed that under most conditions, the reaction rate is at least partially controlled by the rate at which oxygen can be dissolved into the aqueous medium. Thus, although oxygen can be added to the reaction as air, it is preferred to use a relatively pure form of oxygen, and even use elevated pressures. Although no upper limit of oxygen pressure is known oxygen pressures up to 50 atmospheres may be used, and an upper limit of 15 atmospheres is preferred. Agitation is important to maintaining a high oxygen dissolution (hence reaction) rate. Any convenient form of agitation is useful, such as stirring. On the other hand, as is well known to those skilled in the enzyme art, high shear agitation or agitation that produces foam may decrease the activity of the enzyme(s), and should be avoided.

The reaction temperature is an important variable, in that it affects reaction rate and the stability of the enzymes. A reaction temperature of 0°C to 40°C may be used, but the preferred reaction temperature range is from 5°C to 15°C. Operating in the preferred temperature range maximizes recovered enzyme activity at the end of the reaction. The temperature should not be so low that the aqueous solution starts to freeze. Temperature can be controlled by ordinary methods, such as, but not limited to, by using a jacketed reaction vessel and passing liquid of the appropriate temperature through the jacket. The reaction vessel may be constructed of any material that is inert to the reaction ingredients.

Upon completion of the reaction, the enzymes may be removed by filtration or centrifugation and reused. Alternatively, they can be denatured and precipitated by heating, e.g. to 70°C for 5 minutes, and/or can be allowed to remain in the reaction mixture if their presence in the subsequent steps of converting the glyoxylic acid-aminomethylphosphonic acid mixture to N-(phosphonomethyl)glycine, and of recovering N-(phosphonomethyl) glycine from the reaction mixture, is not objectionable.

Following the cessation of contacting the reaction solution with $O_2$, and preferably following the removal of the enzyme glycolate oxidase and the enzyme catalase when present, flavin mononucleotide (FMN) may optionally be removed by contacting the solution with activated carbon. The solution containing glyoxylic acid and aminomethyl-phosphonic acid (which are believed to be in equilibrium with the corresponding imine), is reduced, producing N-(phosphonomethyl)glycine.

Catalytic hydrogenation is a preferred method for preparing N-(phosphonomethyl)glycine from a mixture of glyoxylic acid and aminomethylphosphonic acid. Catalysts suitable for this purpose include (but are not limited to) the various platinum metals, such as iridium, osmium, rhodium, ruthenium, platinum and palladium; also various other transition metals such as cobalt, copper, nickel and zinc. The catalyst may be unsupported, for example as Raney nickel or platinum oxide; or it may be supported, for example as platinum on carbon, palladium on alumina, or nickel on kieselguhr. Palladium on carbon, nickel on kieselguhr and Raney nickel are preferred.

The hydrogenation can be performed at a pH of from 4 to 11, preferably from 5 to 10. Within this pH range, the exact value may be adjusted to obtain the desired pH by adding any compatible, non-interfering base or acid. Suitable bases include, but are not limited to, alkali metal hydroxides, carbonates, bicarbonates and phosphates, while suitable acids include, but are not limited to, hydrochloric, sulfuric, or phosphoric acid.

The hydrogenation temperature and pressure can vary widely. The temperature may generally be in the range of 0°C to 150°C, preferably from 20°C to 90°C, while the $H_2$ pressure is generally in the range of from about atmospheric to about 100 atmospheres, preferably from 1 to 10 atmospheres. The hydrogenation catalyst is employed at a minimum concentration sufficient to obtain the desired reaction rate and total conversion of starting materials under the chosen reaction conditions; this concentration is easily determined by trial. The catalyst may be used in amounts of from 0.001 to 20 or more parts by weight of catalyst per 100 parts of combined weight of the glyoxylic acid and AMPA employed in the reaction.

N-(Phosphonomethyl)glycine, useful as a post-emergent herbicide, may be recovered from the reduced solution, whatever the reducing method employed, by any of the recovery methods known to the art, including those disclosed in the U.S. Patents 4,851,159 and 4,670,191 and in European Patent Applications 186 648 and 413 672.

In the following Examples, which serve to further illustrate the invention, the yields of glyoxylate, formate and oxalate, and the recovered yield of glycolate, are percentages based on the total amount of glycolic acid present at the beginning of the reaction. Analyses of reaction mixtures were performed using high pressure liquid chromatography. Organic acid analyses were performed using a Bio-Rad HPX-87H column, and AMPA and N-(phosphonomethyl)glycine were analyzed using a Bio-Rad Aminex glyphosate analysis column. Reported yields of N-(phosphonomethyl)glycine are based on either glyoxylate or AMPA, depending on which was the limiting reagent in the reaction.

## Example 1

Into a 3 oz. Fischer-Porter glass aerosol reaction vessel was placed a magnetic stirring bar and 10 mL of an aqueous solution containing glycolic acid (0.25 M), aminomethylphosphonic acid (AMPA, 0.263 M), FMN (0.01 mM), propionic acid (HPLC internal standard, 0.125 M), glycolate oxidase (from spinach, 1.0 IU/mL), and catalase (from *Aspergillus niger*, 1,400 IU/mL) at pH 8.5. The reaction vessel was sealed and the reaction mixture was cooled to 15°C, then the vessel was flushed with oxygen by pressurizing to 70 psig and venting to atmospheric pressure five times with stirring. The vessel was then pressurized to 70 psig of oxygen and the mixture stirred at 15°C. Aliquots (0.10 mL) were removed by syringe through a sampling port (without loss of pressure in the vessel) at regular intervals for analysis by HPLC to monitor the progress of the reaction. After 5 h, the HPLC yields of glyoxylate, formate, and oxalate were 70.4 %, 19.6 %, and 2.2 %, respectively, and 5.3 % glycolate remained. The remaining activity of glycolate oxidase and catalase were 27 % and 100 %, respectively, of their initial values.

## Example 2 (Comparative)

The reaction in Example 1 was repeated, using 0.33 M $K_2HPO_4$ in place of 0.265 M AMPA. After 5 h, the HPLC yields of glyoxylate, formate, and oxalate were 34.1 %, 11.1 %, and 0.2 %, respectively, and 58.7 % glycolate remained. After 23 h, the HPLC yields of glyoxylate, formate, and oxalate were 39.4 %, 44.7 %, and 15.34 %, respectively, and no glycolate remained. The remaining activity of glycolate oxidase and catalase were 85 % and 87 %, respectively, of their initial values.

## Example 3 (Comparative)

The reaction in Example 1 was repeated, using 0.263 M bicine buffer in place of 0.265 M AMPA. After 5 h, the HPLC yields of glyoxylate, formate, and oxalate were 42.5 %, 49.6 %, and 10.1 %, respectively, and 0.2 % glycolate remained. The remaining activity of glycolate oxidase and catalase were 47 % and 100 %, respectively, of their initial values.

## Example 4

The reaction in Example 1 was repeated using 5,600 IU/mL catalase from *Aspergillus niger*. After 6 h, the HPLC yields of glyoxylate, formate, and oxalate were 85.5 %, 7.6 %, and 3.3 %, respectively, and 2.5 % glycolate remained. The remaining activity of glycolate oxidase and catalase were 36 % and 100 %, respectively, of their initial values.

## Example 5

The reaction in Example 1 was repeated using 14,000 IU/mL catalase from *Aspergillus niger*. After 6 h, the HPLC yields of glyoxylate, formate, and oxalate were 88.0 %, 3.3 %, and 3.0 %, respectively, and 3.4 % glycolate remained. The remaining activity of glycolate oxidase and catalase were 28 % and 96 %, respectively, of their initial values.

## Example 6

The reaction in Example 1 was repeated using 56,000 IU/mL catalase from *Aspergillus niger*. After 6 h, the HPLC yields of glyoxylate, formate, and oxalate were 84.0 %, 0.4 %, and 2.5 %, respectively, and 8.4 % glycolate remained. The remaining activity of glycolate oxidase and catalase were 16 % and 76 %, respectively, of their initial values.

## Example 7

Into a 3 oz. Fischer-Porter glass aerosol reaction vessel was placed a magnetic stirring bar and 10 mL of an aqueous solution containing glycolic acid (0.25 M), aminomethylphosphonic acid (AMPA, 0.20 M), FMN (0.01 mM), butyric acid (HPLC internal standard, 0.10 M), glycolate oxidase (from spinach, 1.0 IU/mL), and catalase (from *Aspergillus niger*, 14,000 IU/mL) at pH 8.5. The reaction vessel was sealed and the reaction mixture was cooled to 5°C, then the vessel was flushed with oxygen by pressurizing to 70 psig and venting to atmospheric pressure five times with stirring. The vessel was then pressurized to 70 psig of oxygen and the mixture stirred at 5°C. Aliquots (0.10 mL) were removed by syringe through a sampling port (without loss of pressure in the vessel) at regular intervals for analysis by HPLC to monitor the progress of the reaction. After 6 h, the HPLC yields of glyoxylate, formate, and oxalate were 92.3 %, 4.36 %, and 5.5 %, respectively, and no glycolate remained. The remaining activity of glycolate oxidase and catalase were 87 % and 88 %, respectively, of their initial values. The final pH of the reaction mixture was 6.7.

The resulting mixture of glyoxylic acid (0.23 M) and AMPA (0.20 M) was filtered using an Amicon Centriprep 10 concentrator (10,000 molecular weight cutoff) to remove the soluble enzymes, then the filtrate was placed in a 3-oz. Fischer-Porter bottle equipped with a magnetic stirrer bar. To the bottle was then added 0.100 g of 10% Pd/C and the bottle sealed, flushed with nitrogen gas, then pressurized to 50 psi with hydrogen and stirred at 25°C. After 17 h, the concentration of N-(phosphonomethyl)-glycine (determined by HPLC) was 0.13 M (66% yield based on AMPA).

Example 8

Into a 3 oz. Fischer-Porter glass aerosol reaction vessel was placed a magnetic stirring bar and 10 mL of an aqueous solution containing glycolic acid (0.50 M), aminomethylphosphonic acid (AMPA, 0.40 M), FMN (0.01 mM), butyric acid (HPLC internal standard, 0.10 M), glycolate oxidase (from spinach, 1.0 IU/mL), and catalase (from *Aspergillus niger*, 14,000 IU/mL) at pH 8.5. The reaction vessel was sealed and the reaction mixture was cooled to 5°C (instead of 15°C as described in previous examples), then the vessel was flushed with oxygen by pressurizing to 70 psig and venting to atmospheric pressure five times with stirring. The vessel was then pressurized to 70 psig of oxygen and the mixture stirred at 5°C. Aliquots (0.10 mL) were removed by syringe through a sampling port (without loss of pressure in the vessel) at regular intervals for analysis by HPLC to monitor the progress of the reaction. After 17.5 h, the HPLC yields of glyoxylate, formate, and oxalate were 91.0%, 2.9%, and 2.9%, respectively, and 4.1 % glycolate remained. The final pH of the reaction mixture was 6.7. The remaining activity of glycolate oxidase was 63% and 91%, respectively, of their initial value.

The resulting mixture of glyoxylic acid (0.46 M) and AMPA (0.40 M) was filtered using an Amicon Centriprep 10 concentrator (10,000 molecular weight cutoff) to remove the soluble enzymes, then the filtrate was placed in a 3-oz. Fischer-Porter bottle equipped with a magnetic stirrer bar. To the bottle was then added 0.100 g of 10% Pd/C and the bottle sealed, flushed with nitrogen gas, then pressurized to 50 psi with hydrogen and stirred at 25°C. After 17 h, the concentration of N-(phosphonomethyl)glycine (determined by HPLC) was 0.29 M (72% yield based on AMPA).

Example 9

The enzymatic oxidation of glycolic acid in Example 8 was repeated, using 10 mL of an aqueous solution containing glycolic acid (0.75 M), aminomethylphosphonic acid (AMPA, 0.60 M), FMN (0.01 mM), butyric acid (HPLC internal standard, 0.10 M), glycolate oxidase (from spinach, 2.0 IU/mL), and catalase (from *Aspergillus niger*, 14.000 IU/mL) at pH 8.5. After 40 h, the HPLC yields of glyoxylate, formate, and oxalate were 83.2%, 2.3%, and 7.5%, respectively, and no glycolate remained. The final pH of the reaction mixture was 6.8. The remaining activity of glycolate oxidase and catalase were 65 % and 86 %, respectively, of their initial values.

The resulting mixture of glyoxylic acid (0.62 M) and AMPA (0.60 M) was filtered using an Amicon Centriprep 10 concentrator (10,000 molecular weight cutoff) to remove the soluble enzymes, then the filtrate was placed in a 3-oz. Fischer-Porter bottle equipped with a magnetic stirrer bar. To the bottle was then added 0.100 g of 10% Pd/C and the bottle sealed, flushed with nitrogen gas, then pressurized to 50 psi with hydrogen and stirred at 25°C. After 24 h, the concentration of N-(phosphonomethyl)glycine (determined by HPLC) was 0.42 M (70% yield based on AMPA).

Example 10

The enzymatic oxidation of glycolic acid in Example 8 was repeated, using 10 mL of an aqueous solution containing glycolic acid (1.0 M), aminomethylphosphonic acid (AMPA, 0.80 M), FMN (0.01 mM), butyric acid (HPLC internal standard, 0.10 M), glycolate oxidase (from spinach, 2.0 IU/mL), and catalase (from *Aspergillus niger*, 14,000 IU/mL) at pH 8-5. After 66 h, the HPLC yields of glyoxylate, formate, and oxalate were 78.9%, 2.2%, and 12.1%, respectively, and 2.0% glycolate remained. The final pH of the reaction mixture was 6.9. The remaining activity of glycolate oxidase and catalase were 64 % and 87 %, respectively, of their initial values.

The resulting mixture of glyoxylic acid (0.79 M) and AMPA (0.80 M) was filtered using an Amicon Centriprep 10 concentrator (10,000 molecular weight cutoff) to remove the soluble enzymes, then the filtrate was placed in a 3-oz. Fischer-Porter bottle equipped with a magnetic stirrer bar. To the bottle was then added 0.100 g of 10% Pd/C and the bottle sealed, flushed with nitrogen gas, then pressurized to 50 psi with hydrogen and stirred at 25°C. After 23 h, the concentration of N-(phosphonomethyl)glycine (determined by HPLC) was 0.51 M (65% yield based on glyoxylic acid).

## Example 11

The reaction in Example 8 was repeated at pH 8.0. After 17.5 h, the HPLC yields of glyoxylate, formate, and oxalate were 87.0 %, 2.2 %, and 1.9 %, respectively, and 8.5 % glycolate remained. The remaining activity of glycolate oxidase and catalase were 44 % and 97 %, respectively, of their initial values.

## Example 12

The reaction in Example 8 was repeated at pH 7. After 17.5 h, the HPLC yields of glyoxylate, formate, and oxalate were 88.0 %, 1.4 %, and 1.9 %, respectively, and 8.2 % glycolate remained. The remaining activity of glycolate oxidase and catalase were 44 % and 93 %, respectively, of their initial values.

## Example 13

Into a 3 oz. Fischer-Porter glass aerosol reaction vessel was placed a magnetic stirring bar and 10 mL of an aqueous solution containing glycolic acid (0.50 M), FMN (0.01 mM), isobutyric acid (HPLC internal standard, 0.10 M), glycolate oxidase (from spinach, 1.0 IU/mL), and catalase (from *Aspergillus niger*, 14,000 IU/mL) at pH 8.5. The reaction vessel was sealed and the reaction mixture was cooled to 5°C, then the vessel was flushed with oxygen by pressurizing to 70 psig and venting to atmospheric pressure five times with stirring. The vessel was then pressurized to 70 psig of oxygen and the mixture stirred at 5°C. Aliquots (0.10 mL) were removed by syringe through a sampling port (without loss of pressure in the vessel) at regular intervals for analysis by HPLC to monitor the progress of the reaction. After 21 h, the HPLC yields of glyoxylate, formate, and oxalate were 81.7 %, 1.2 %, and 2.2 %, respectively, and 7.5 % glycolate remained. The remaining activity of glycolate oxidase and catalase were 19 % and 77%, respectively, of their initial values. This reaction was then repeated with 0.50 M glycolic acid and 0.25 M, 0.375 M, 0.40 M, 0.50 M, or 0.625 M aminomethylphosphonic acid (AMPA) present, and the yields of reaction products and enzyme recoveries for these reactions are listed below:

| [AMPA] (M) | glyoxylate (%) | formate (%) | oxalate (%) | glycolate (%) | glycolate oxidase(%) | catalase (%) |
|---|---|---|---|---|---|---|
| 0.00 | 81.7 | 1.2 | 2.2 | 7.5 | 19 | 77 |
| 0.25 | 79.4 | 2.1 | 3.3 | 2.5 | 48 | 79 |
| 0.375 | 78.3 | 2.3 | 3.6 | 1.7 | 57 | 95 |
| 0.40 | 91.0 | 2.9 | 2.9 | 4.1 | 63 | 91 |
| 0.50 | 85.2 | 1.5 | 3.3 | 5.5 | 49 | 93 |
| 0.625 | 79.6 | 1.7 | 1.8 | 14.0 | 42 | 94 |

## Example 14

The enzymatic oxidation of glycolic acid in Example 8 was repeated, using 10 mL of an aqueous solution containing glycolic acid (0.25 M), aminomethylphosphonic acid (AMPA, 0.263 M), FMN (0.01 mM), butyric acid (HPLC internal standard, 0.25 M), glycolate oxidase (from spinach, 1.0 IU/mL), and catalase (from *Aspergillus niger*, 14,000 IU/mL) at pH 7.0 and 15°C. After 8 h, the HPLC yields of glyoxylate, formate, and oxalate were 82.8%, 0.9%, and 2.1%, respectively, and 13.9% glycolate remained. The final pH of the reaction mixture was 6.6.

This mixture of glyoxylic acid (0.21 M) and AMPA (0.263 M) was filtered using an Amicon Centriprep 10 concentrator (10,000 molecular weight cutoff) to remove the soluble enzymes, then the filtrate and 50 mg of 10% Pd/C were placed in a stainless steel pressure vessel equipped with glass liner. The vessel was sealed, flushed with nitrogen gas, then pressurized to 1000 psi with hydrogen gas and shaken at 25°C. The pressure in the vessel fell to a stable value in the first 0.5 h of reaction, and the vessel was then repressurized to 1000 psi. After 4 h, the pressure in the vessel was vented, and the vessel flushed with nitrogen. The concentration of N-(phosphono-methyl)glycine (determined by HPLC) was 0.16 M (76% yield based on glyoxylic acid).

Example 15

The enzymatic oxidation of glycolic acid in Example 14 was repeated at pH 8. After 8 h, the HPLC yields of glyoxylate, formate, and oxalate were 86.7%, 1.8%, and 4.1%, respectively, and 13.2% glycolate remained. The final pH of the reaction mixture was 6.7.

This mixture of glyoxylic acid (0.22 M) and AMPA (0.263 M) was hydrogenated at 1000 psi using the same procedure as described in Example 5. After 4 h, the concentration of N-(phosphonomethyl)glycine (determined by HPLC) was 0.14 M (64% yield based on glyoxylic acid).

Example 16

The enzymatic oxidation of glycolic acid in Example 14 was repeated at pH 9. After 7 h, the HPLC yields of glyoxylate, formate, and oxalate were 70.0%, 5.6%, and 11.1%, respectively, and no glycolate remained. The final pH of the reaction mixture was 6.8.

This mixture of glyoxylic acid (0. 18 M) and AMPA (0.263 M) was hydrogenated at 1000 psi using the same procedure as described in Example 5. After 4 h, the concentration of N-(phosphonomethyl)glycine (determined by HPLC) was 0.094 M (52% yield based on AMPA).

Example 17

The enzymatic oxidation of glycolic acid in Example 14 was repeated at pH 8.5, and using initial concentrations of glycolic acid and AMPA of 0.50 M and 0.40 M, respectively. After 16.5 h, the HPLC yields of glyoxylate, formate, and oxalate were 85.4%, 3.5%, and 6.3%, respectively, and 1.4% glycolate remained. The final pH of the reaction mixture was 7.0.

This mixture of glyoxylic acid (0.43 M) and AMPA (0.40 M) was hydrogenated at 1000 psi using the same procedure as described in Example 5. After 4 h, the concentration of N-(phosphonomethyl)glycine (determined by HPLC) was 0.30 M (75% yield based on AMPA).

Example 18

The enzymatic oxidation of glycolic acid in Example 8 was repeated, using 10 mL of an aqueous solution containing glycolic acid (0.50 M), aminomethylphosphonic acid (AMPA, 0.375 M), FMN (0.01 mM), butyric acid (HPLC internal standard, 0.10 M), glycolate oxidase (from spinach, 1.0 IU/mL), and catalase (from *Aspergillus niger*, 14,000 IU/mL) at pH 8.5. After 17 h, the HPLC yields of glyoxylate, formate, and oxalate were 87.1%, 1.9%, and 2.1%, respectively, and 8.9% glycolate remained. The final pH of the reaction mixture was 6.7.

The resulting mixture of glyoxylic acid (0.435 M) and AMPA (0.375 M) was filtered using an Amicon Centriprep 10 concentrator (10,000 molecular weight cutoff) to remove the soluble enzymes, then the filtrate was mixed with 50 mg of decolorizing carbon (to remove FMN) and again filtered. The resulting filtrate was placed in a 3-oz. Fischer-Porter bottle equipped with a magnetic stirrer bar. To the bottle was then added 0.100 g of 10% Pd/C and the bottle sealed, flushed with nitrogen gas, then pressurized to 50 psi with hydrogen and stirred at 25°C. After 17 h, the concentration of N-(phosphonomethyl)glycine (determined by HPLC) was 0.372 M (99% yield based on AMPA).

**Claims**

1. A process for preparing N-(phosphonomethyl)glycine which comprises hydrogenating a mixture, wherein said mixture is enzymatically produced by reacting glycolic acid and oxygen in an aqueous solution containing aminomethylphosphonic acid and the enzymes glycolate oxidase and catalase.

2. An improved process for preparing N-(phosphonomethyl)glycine comprising the step of reducing a mixture of glyoxylic acid and aminomethylphosphonic acid by hydrogenation; said mixture being enzymatically generated *in situ* in an aqueous solution by incorporating into the aqueous solution glycolic acid, a first catalyst adapted to catalyze the oxidation of glycolic acid with oxygen to glyoxylic acid and hydrogen peroxide, and a second catalyst adapted to catalyze the decomposition of hydrogen peroxide, adjusting the pH of the solution to between 6 and about 10, contacting the solution with a source of oxygen at an effective temperature and sufficient time to convert at least a portion of the glycolic component to the glyoxylic component in the presence of aminomethylphosphonic acid, and ceasing contacting the solution with oxygen prior to the reducing step.

3. The process of Claim 2 wherein the first catalyst is glycolate oxidase and the second catalyst is catalase.

4. The process of Claim 1 wherein the hydrogenating is performed in the presence of a hydrogenation catalyst.

5. The process of Claim 2 wherein the reducing is performed in the presence of a hydrogenation catalyst.

6. The process of Claim 4 or 5 wherein the hydrogenation catalyst is selected from the group consisting of palladium on carbon, nickel on kieselguhr and Raney nickel.

7. The process of Claim 6 wherein the hydrogenation catalyst is present in an amount of from 0.001 to 20 parts by weight catalyst per 100 parts of combined weight of the glyoxylic acid and aminomethylphosphonic acid employed.

8. The process of Claim 7 wherein the hydrogenation is performed at a pH of from 4 to 11 within a temperature range of 0°C to 150°C and at a hydrogen pressure of 1 to about 100 atmospheres.

9. The process of Claim 7 wherein the hydrogenation is performed at a pH of from 5 to 10 within a temperature range of 20°C to 90°C and at a hydrogen pressure of 1 to about 10 atmospheres.

10. The product of a process according to any preceding claim.

**European Patent Office**

## EUROPEAN SEARCH REPORT

Application Number

EP    92 31 0064

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | WO-A-9 105 868 (DU PONT DE NEMOURS) 2 May 1991 * page 6 - page 13; claims * | 1-9 | C12P7/40 C12P13/04 C07F9/38 |
| D,Y | EP-A-0 186 648 (MONSANTO) 2 July 1986 * page 10, column 11 - page 14 * | 1-9 | |
| D,X | | 10 | |
| A | FR-A-2 294 182 (MONSANTO) 9 July 1976 * page 3; claims * | 1 | |
| X | | 10 | |
| D | & US-A-4 094 928 | | |
| A | FR-A-2 294 181 (MONSANTO) 9 July 1976 * claims * | 1 | |
| X | | 10 | |

TECHNICAL FIELDS SEARCHED (Int. Cl.5)

C12P
C07F

The present search report has been drawn up for all claims

| Place of search | Date of completion of the search | Examiner |
|---|---|---|
| THE HAGUE | 22 FEBRUARY 1993 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)